# EUROPEAN PATENT APPLICATION

(11) **EP 1 566 097 A1**
(43) Date of publication of application: **24.08.2005**
(21) Application number: 03811882.4
(22) Date of filing: 06.11.2003
(51) Int. Cl.: A01H 1/00, C12N 15/09

(54) **METHOD OF IN PLANTA TRANSFORMATION OF KENAF PLANT WITH AGROBACTERIUM TUMEFACIENS**

(30) Priority: 25.11.2002 JP 2002340992
(71) Applicant: KUMIAI CHEMICAL INDUSTRY CO., LTD., Tokyo 110-8782 (JP); Kojima, Mineo, Ueda-shi, Nagano 386-0018 (JP)
(72) Inventor: KOJIMA, Mineo, Ueda-shi, Nagano 386-0018 (JP); NOZUE, Masayuki, Chiisagata-gun, Nagano 389-0505 (JP); SHIOIRI, Hidenari, Ueda-shi, Nagano 386-1213 (JP); NOGAWA, Masahiro, Ueda-shi, Nagano 386-0017 (JP); KIGUCHI, Kenji, Ueda-shi, Nagano 386-1327 (JP); SHIMIZU, Tsutomu, Ogasa-gun, Shizuoka 439-0035 (JP)
(74) Representative: Tollervey, Rebecca Marie
(86) International application number: PCT/JP2003/014148
(87) International publication number: WO 2004/047522

(57) **Abstract**

The present invention provides an *in planta* transformation method of kenaf plants, which is **characterized in that** wounds on the axillary buds of a kenaf plant are inoculated with *Agrobacterium tumefaciens*.

## Description

### Technical Field

The present invention relates to an *in planta* transformation method of kenaf plants using *Agrobacterium tumefaciens*.

### Background Art

In recent years, kenaf plants have attracted much attention in the following respects (Sellers T and Reichert N, "Kenaf: properties, processing and products," U.S.A., Mississippi Univ. Press, 1999). First, kenaf plants grow very rapidly. Second, high-quality fibers are produced from kenaf plants. For these reasons, kenaf plants can absorb a large amount of CO₂ in the air during its growth, and can be substituted for trees in forest in the production of pulp. Thus, kenaf plants are regarded as plants that can contribute to protect the earth from global warming.

As a common plant transformation method, a method comprising introducing a foreign gene into callus or tissue culture cells and then regenerating them into a plant body has been known (Hooykaas PJJ and Schilperoort RA, *Agrobacterium* and Plant Genetic Engineering, "Plant Molecular Biology," U.S.A., 1992, vol. 19, pp. 15-38; and Zupan JR and Zambryski P, Transfer of T-DNA from *Agrobacterium* to the Plant Cell, "Plant Physiology," U.S.A., 1995, vol. 107, pp. 1041-1047). However, such transformation methods have several disadvantages. First, the methods are disadvantageous in that they require sterile conditions. Second, it takes a long period of time to carry out these methods. Third, somatic mutation or somaclonal variation occurs frequently in plant cells during tissue culture. Moreover, some plants are recalcitrant to regeneration by these methods. In order to solve the aforementioned problems involved in the conventional transformation methods, an *in planta* transformation method has been developed (Birch R. G., Plant Transformation - Problems and Strategies for Practical Application, "Annual Review of Plant Physiology and Plant Molecular Biology," U.S.A., 1997, vol. 48, pp. 297-326). The "*in planta* transformation method" is a method for transforming cells of a growing plant without performing the aforementioned tissue culture.

Previously, the present inventors have developed a simple and efficient *in planta* transformation method of buckwheat plants (*Fagopyrum esculentum*) using *Agrobacterium tumefaciens* (Kojima M. et al., Development of a Simple and Efficient Method for Transformation of Buckwheat Plants (*Fagopyrum esculentum*) Using *Agrobacterium tumefaciens*, "Bioscience, Biotechnology, and Biochemistry," 2000, vol. 64, pp. 845-847). In this method, apical meristems of seedlings of buckwheat are perforated with a needle and then inoculated with *Agrobacterium tumejaciens*. The term "apical meristems" are used herein to mean tissues, which exist at the tip of the growth axis of a plant and wherein cells are growing.

On the other hand, as a method for transforming kenaf plants, a method involving inoculating *Agrobacterium tumefaciens* having a foreign gene onto a fragment of leaf tissue or incorporating a tungsten or gold particle carrying a foreign gene on the surface thereof into a fragment of leaf tissue has been disclosed (US Patent No. 5,998,207). This method is a common plant transformation method as described above. However, this method is problematic in that it has poor workability. The aforementioned *in planta* transformation method of buckwheat plants (*Fagopyrum esculentum*) using *Agrobacterium tumefaciens* (Kojima M. et al., Development of a Simple and Efficient Method for Transformation of Buckwheat Plants (*Fagopyrum esculentum*) Using *Agrobacterium tumefaciens*, "Bioscience, Biotechnology, and Biochemistry," 2000, vol. 64, pp. 845-847) was applied to kenaf plants. However, the plants could not be transformed efficiently. Thus, an *in planta* transformation method of kenaf plants has not yet been established.

### Disclosure of the Invention

It is an object of the present invention to provide an *in planta* transformation method of kenaf plants, which can simply and efficiently be carried out.

As a result of intensive studies directed towards achieving the aforementioned object, the present inventors have found that a kenaf plant is efficiently transformed by inoculating *Agrobacterium tumefaciens* into wounds on the axillary buds of the kenaf plant, thereby completing the present invention.

That is to say, the present invention relates to an *in planta* transformation method of kenaf plants, which is characterized in that wounds on the axillary buds of a kenaf plant are inoculated with *Agrobacterium tumefaciens*.

The present invention also relates to the above described *in planta* transformation method of kenaf plants, which comprises a step of cutting off the upper part of stem of a kenaf plant and allowing the axillary buds to appear.

The present invention further relates to the above described *in planta* transformation method of kenaf plants, which comprises a step of wounding the above described axillary buds.

The present invention further relates to the above described *in planta* transformation method of kenaf plants, wherein the above described wound is a perforated hole.

The present invention further relates to the above described *in planta* transformation method of kenaf plants, wherein *Agrobacterium tumefaciens* to be inoculated is an *Agrobacterium tumefaciens* M-21 mutant.

The present invention further relates to the above described *in planta* transformation method of kenaf plants, wherein the above described inoculation is carried out by applying an *Agrobacterium tumefaciens* suspension to the wound.

The present invention will be described in detail below.

The *in planta* transformation method of the present invention comprises: a step of allowing the axillary buds of a kenaf plant to appear; a step of wounding the appearing axillary buds; a step of inoculating the wounds with *Agrobacterium tumefaciens*; and a step of eliminating all the axillary buds except for a single axillary bud after the plant has grown.

Target plants to which the *in planta* transformation method of the present invention is applied are kenaf plants. Any type of kenaf plant can be used herein. *Hibiscus cannabinus* var. Aokawa No. 3 is an example of such a kenaf plant. In the *in planta* transformation method of the present invention, for example, a young kenaf plant with a height of approximately 70 cm is preferably used. Conditions for the growth of a kenaf plant from sowing to a mature plant consist of, for example, an illumination of 30,000 lux, a temperature between 20°C and 25°C, and a photoperiod consisting of 10 hours of photophase and 14 hours of scotophase.

In the *in planta* transformation method of the present invention, the axillary buds are first allowed to appear from a kenaf plant. Preferably, multiple the axillary buds are allowed to appear from a single kenaf plant. "The axillary buds" are a certain type of lateral buds, which generate towards the lateral side of the stem axis of a plant and are formed with leaf sap. The upper part of the stem of a kenaf plant is cut off, and the bottom part thereof is left with the remaining 6 or 7 leaves. Thereafter, the plant is allowed to grow, so that the axillary buds can be allowed to appear at the junction sites of the stem and the petioles. When the in *planta* transformation method of the present invention is applied to the axillary buds that have come out not artificially but naturally during the growth of a kenaf plant, the aforementioned step is not necessary.

Subsequently, the axillary buds obtained by the aforementioned step are wounded. Any type of wound may be made herein, as long as it can be infected by inoculation of *Agrobacterium tumefaciens*. Examples of such a wound may include a perforated hole made on an axillary bud and an incision made thereon. A needle (φ 0.71 mm) can be used as a device for perforating the axillary buds, for example. The number of perforated holes is preferably 2 or 3 per axillary bud. On the other hand, as a device for incising the axillary buds, cutting devices such as a knife or cutter can be used, for example. The site to be wounded is preferably a junction site of the petiole of an axillary bud. When the *in planta* transformation method of the present invention is applied to wounds that are not artificially but naturally made, this step is not necessary.

Subsequently, the resulting wounds on the axillary buds are inoculated with *Agrobacterium tumefaciens*. In general, *Agrobacterium tumefaciens* is infected onto a plant to form a tumor known as crown gall. This is because a region known as T-DNA region located on a Ti plasmid in *Agrobacterium tumefaciens* is transferred into the plant during the infection and it is incorporated into the genome of the plant. For example, a foreign gene to be incorporated into the genome of a kenaf plant is inserted into a T-DNA region on a Ti plasmid. Thereafter, the kenaf plant is infected with *Agrobacterium tumefaciens* having this T-DNA region, so that the above described foreign gene can be incorporated into the genome of the kenaf plant. Any gene encoding a protein or peptide can be used as such a foreign gene.

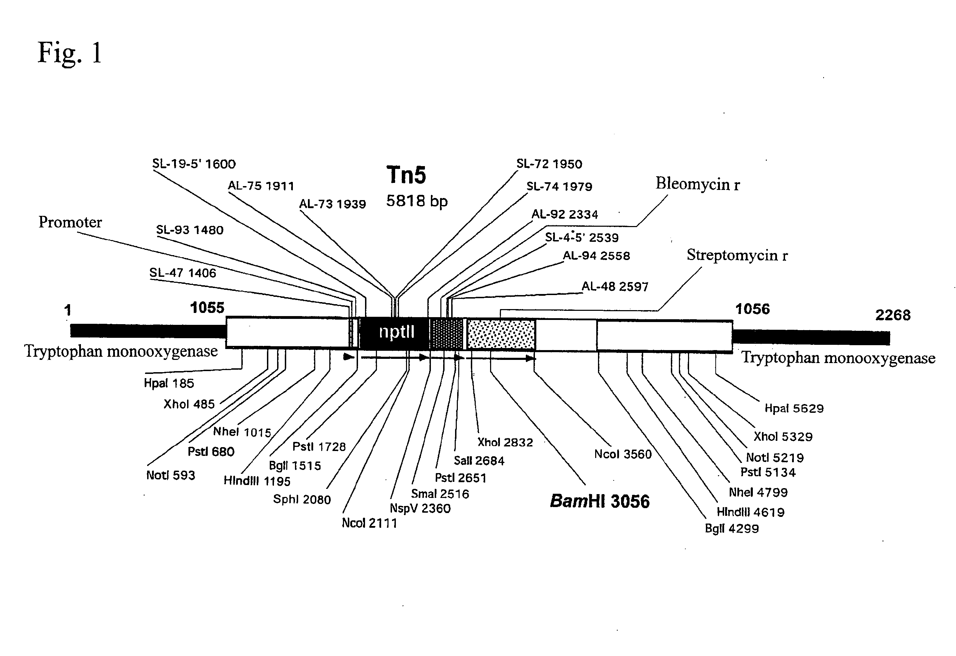

In the *in planta* transformation method of the present invention, any strain of *Agrobacterium tumefaciens* can be used herein as *Agrobacterium tumefaciens* to be inoculated herein, as long as it can be infected into kenaf plants. A nonpathogenic *Agrobacterium tumefaciens* M-21 mutant is preferable. The *Agrobacterium tumefaciens* M-21 mutant is obtained by mutating a pathogenic *Agrobacterium tumefaciens* A208 strain (C58 chromosome, nopaline type, T37pTi) by transposon 5 (Tn5) mutation (Majumder P. et al., J Biosci Bioeng 90: 328-331, 2000). Figure 1 shows a structure of the site into which a Tn5 has been inserted in the T-DNA region of *Agrobacterium tumefaciens* M-21 mutant. As shown in Figure 1, in *Agrobacterium tumefaciens* M-21 mutant, a Tn5 has been inserted into a tryptophan monooxygenase gene (hereinafter referred to as an "*iaaM* gene"), which is involved in the biosynthesis of indoleacetic acid (IAA), located in the T-DNA region of a Ti plasmid. *Agrobacterium tumefaciens* M-21 mutant has an ability to incorporate its T-DNA region into the chromosome of a host, but it does not form a crown gall.

Any method of inoculating wounds on the axillary buds with *Agrobacterium tumefaciens* can be applied, as long as it is capable of infecting meristems with *Agrobacterium tumefaciens* via wounds on the axillary buds. Examples of such a method may include a method of applying an *Agrobacterium tumefaciens* suspension to wounds on the axillary buds using a cotton swab that has been immersed in the suspension, a method of directly injecting an *Agrobacterium tumefaciens* suspension into wounds on the axillary buds using a pipette or syringe, and a method of spraying an *Agrobacterium tumefaciens* suspension to wounds on the axillary buds using a nebulizer. An *Agrobacterium tumefaciens* suspension is prepared by the following method. First, *Agrobacterium tumefaciens* is subjected to shake culture in LB liquid medium containing kanamycin (50 µg/ml) and rifampicin (10 µg/ml) at 28°C for 18 hours. Thereafter, the culture product is centrifuged to recover *Agrobacterium tumefaciens*, followed by washing with water. Thereafter, *Agrobacterium tumefaciens* is suspended in water, resulting in a concentration of 1.0 × 10⁸ cells/ml or higher. The obtained suspension is defined as an *Agrobacterium tumefaciens* suspension.

Subsequently, the kenaf plant inoculated with *Agrobacterium tumefaciens* as described above (hereinafter referred to as a "transformed kenaf plant body") is allowed to grow for 3 or 4 days. By allowing the transformed kenaf plant body to grow for 3 or 4 days, the axillary buds become approximately 3 cm long.

After the transformed kenaf plant body has been allowed to grow for 3 or 4 days, one is selected from the axillary buds inoculated with *Agrobacterium tumefaciens*, and all the remaining axillary buds are eliminated. The transformed kenaf plant body is then allowed to grow until it becomes a mature plant. An axillary bud located in the uppermost position among multiple the axillary buds is preferably selected as one to be left.

It can be confirmed by the PCR method, the Southern hybridization method, or the Northern hybridization method, whether or not a T-DNA region derived from *Agrobacterium tumefaciens* has been incorporated into the genome of the transformed kenaf plant body. For example, DNA is prepared from the transformed kenaf plant body, and DNA-specific primers are designed. Using the primers, PCR is carried out. Thereafter, the obtained PCR product is subjected to agarose gel electrophoresis, polyacrylamide gel electrophoresis, or capillary electrophoresis, and then stained with ethidium bromide, SYBR green, or the like. Thus, the PCR product is detected as a single band, and it is confirmed that the T-DNA region derived from *Agrobacterium tumefaciens* has been incorporated in the plant genome. Alternatively, PCR is carried out using primers that have previously been labeled with a fluorescent dye, so as to detect a PCR product. Alternatively, a method comprising allowing a PCR product to bind to a solid phase such as a microplate and then confirming the PCR product by a fluorescent reaction, enzyme reaction, or the like, may also be adopted.

The above described *in planta* transformation method of the present invention is applied, so as to solve the aforementioned problems of the conventional transformation methods. Moreover, by the *in planta* transformation method of the present invention, a transformed kenaf plant body expressing a desired foreign gene can be obtained simply and efficiently.

When *Agrobacterium tumefaciens* M-21 mutant is used in the *in planta* transformation method of the present invention, the transformed kenaf plant body exhibits a phenotype wherein the stem is thick. In *Agrobacterium tumefaciens* M-21 mutant, only the *iaaM* gene is mutated, and all other genes including genes that are associated with the biosynthesis of cytokinin existing in the T-DNA region are not mutated. Accordingly, it is considered that the kenaf plant body transformed by *Agrobacterium tumefaciens* M-21 mutant synthesizes a high level of cytokinin. Thus, it is considered that such a high level of cytokinin causes hormone imbalance and that the transformed kenaf plant thereby exhibits a phenotype such a thick stem. Hence, because the kenaf plant bodies transformed by *Agnobacterizrna tumefaciens* M-21 mutant exhibit a thick stem as a phenotype, a large amount of material can industrially be obtained from the individual transformed kenaf plant body. For such a reason, the aforementioned transformed kenaf plant body can be a good material for production of pulp, for example. Alternatively, since the aforementioned transformed kenaf plant body has a thick stem, it can survive as a tree that is robust and resistant to a natural disaster. Thus, it is considered that the aforementioned transformed kenaf plant bodies can be substituted for trees in forest, when the plant bodies are forested.

On the other hand, such transformed kenaf plant bodies are allowed to self-pollinate to set seeds. Thus, seeds of the next generation that inherit genotypes from the transformed kenaf plant bodies can be obtained.

This specification includes part or all of the contents as disclosed in the specification and/or drawings of JP Application No. 2002-340992, which is a priority document of the present application.

### Brief Description of the Drawings

Figure 1 shows a structure of the site into which a Tn5 has been inserted in the T-DNA region of *Agrobacterium tumefaciens* M-21 mutant;
Figure 2 shows photographs of each stem of transformed plant bodies (T₀) and non-transformed plant bodies (T₀), which were taken 1 year after the transformation;
Figure 3 shows photographs of transformed plant bodies (T₁) and non-transformed plant bodies (T₁), which were taken 2 months after the sowing of seeds;

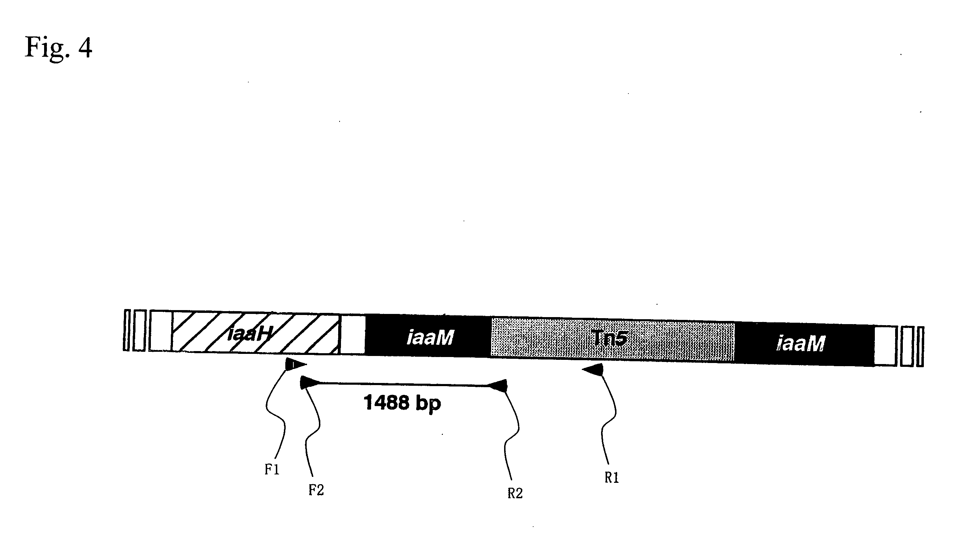

Figure 4 shows a DNA fragment (1,488 bp) that spans a tryptophan monooxygenase gene and Tn5 that are located in T-DNA region on Ti plasmid of *Agrobacterium tumefaciens* M-21 mutant, and locations corresponding to primers used in nested PCR for amplifying the above DNA fragment. In the figure, F1 represents the location of a forward primer used for the first PCR, R1 represents the location of a reverse primer used for the first PCR, F2 represents the location of a forward primer used for the second PCR, and R2 represents the location of a reverse primer used for the second PCR. Also, in the figure, *iaaH* represents indole acetoamide hydrolase, and *iaaM* represents a tryptophan monooxygenase gene;
Figure 5 shows photographs indicating the results of agarose electrophoresis performed on respective PCR products derived from non-transformed plant bodies (T₀) and transformed plant bodies (T₀); and
Figure 6 shows photographs indicating the results of agarose electrophoresis performed on respective PCR products derived from non-transformed plant bodies (T₁) and transformed plant bodies (T₁).

### Best Mode for Canying Out the Invention

### [Examples]

The present invention will be further specifically described in the following examples. However, the examples are not intended to limit the technical scope of the present invention.

### Example 1. In planta transformation of kenaf plant with Agrobacterium tumefaciens

### (1) Growing of kenaf plant

Kenaf plant (*Hibiscus cannabinus* var. Aokawa No. 3) seeds were sown on soils in two pots (each 4 seeds; φ 23 cm; Akadama soil: vermiculite = 1:1), and they were allowed to grow under conditions consisting of an illumination of 30,000 lux, a temperature between 20°C and 25°C, and a photoperiod consisting of 10 hours of photophase and 14 hours of scotophase. Three months after the sowing, kenaf plants with a height of approximately 70 cm were used for transformation.

### (2) Preparation of Agrobacterium tumefaciens

*Agrobacterium tumefaciens* M-21 mutant was cultured in LB medium containing kanamycin (50 µg/ml) and rifampicin (10 µg/ml) at 28°C for 18 hours. Thereafter, the cell mass was recovered by centrifugation, and it was then washed with water. After washing, the cell mass was suspended in water, resulting in a concentration of 1.0 × 10⁸ cells/ml. The thus obtained suspension was defined as an inoculum suspension.

### (3) In planta transformation of kenaf plant with Agrobacterium tumefaciens

The upper part of the stem (35 cm) of the aforementioned kenaf plant was cut off, and approximately 35 cm of the bottom part thereof was left with remaining 6 or 7 leaves. At this stage, no the axillary buds were observed at the junction sites of the petioles and the stem. This plant was allowed to grow for 3 days, until the axillary buds were observed at the junction sites of the petioles and the stem. Two or three the axillary buds appearing in the upper part of the stem were perforated with a needle (φ 0.71 mm) at two or three points for each axillary bud, so as to create perforated holes. Thereafter, perforated holes on the axillary buds of the plant in one pot were inoculated with *Agrobacterium tumefaciens* M-21 mutant, using a cotton swab that has been immersed in the aforementioned inoculum suspension (hereinafter referred to as a "transformed plant body (T₀)").

On the other hand, as a control, perforated holes on the axillary buds of the other kenaf plant were inoculated with water, using a cotton swab that has been immersed in water (hereinafter referred to as a "non-transformed plant body (T₀)").

Thereafter, both the transformed plant body (T₀) and the non-transformed plant body (T₀) were allowed to grow for 3 or 4 days, until the axillary buds had a length of approximately 3 cm. After the plant bodies had been allowed to grow for 3 or 4 days, one axillary bud located in the uppermost position was left, and all other the axillary buds were eliminated. Thereafter, the transformed plant body (T₀) and the non-transformed plant body (T₀) were allowed to grow until they became mature plants. Thereafter, both the transformed plant body (T₀) and the non-transformed plant body (T₀) were allowed to self-pollinate to set seeds, respectively. Subsequently, seven seeds were randomly selected from seeds obtained from each of the transformed plant body (T₀) and the non-transformed plant body (T₀), respectively, and the selected seeds were allowed to grow under the growth conditions described in (1) above. Hereinafter, the thus grown plant bodies are referred to as transformed plant bodies (T₁) and non-transformed plant bodies (T₁), respectively.

### Example 2. Difference between the phenotype of transformed kenaf plant body and that of non-transformed kenaf plant body

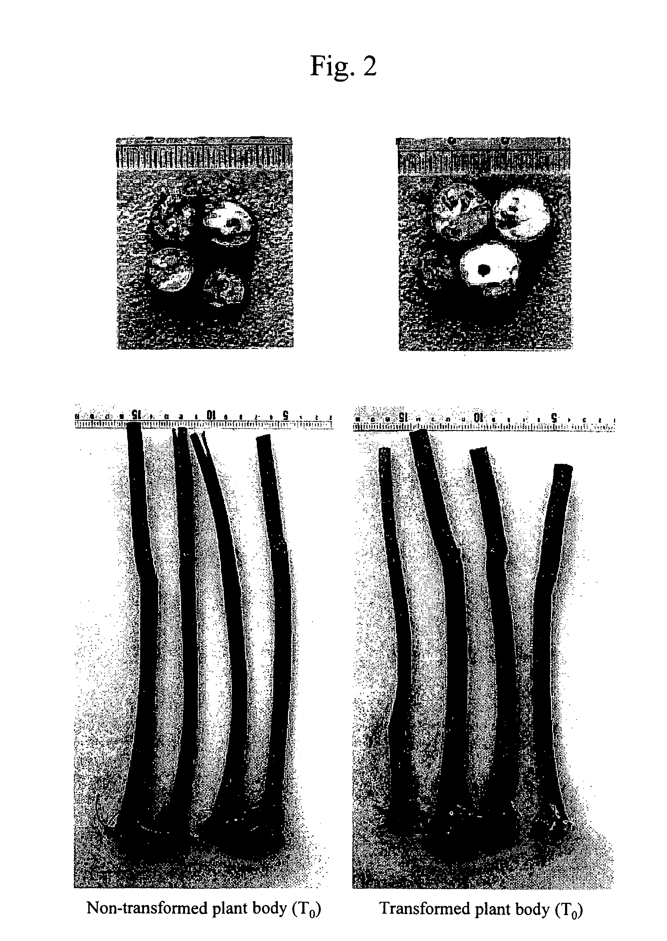

Figure 2 shows photographs of the stems of the transformed plant bodies (T₀) and those of the non-transformed plant bodies (T₀), which were taken 1 year after the transformation. In addition, the thickness of the stem of the above transformed plant body (T₀) and that of the above non-transformed plant body (T₀) are shown in the following Table 1.

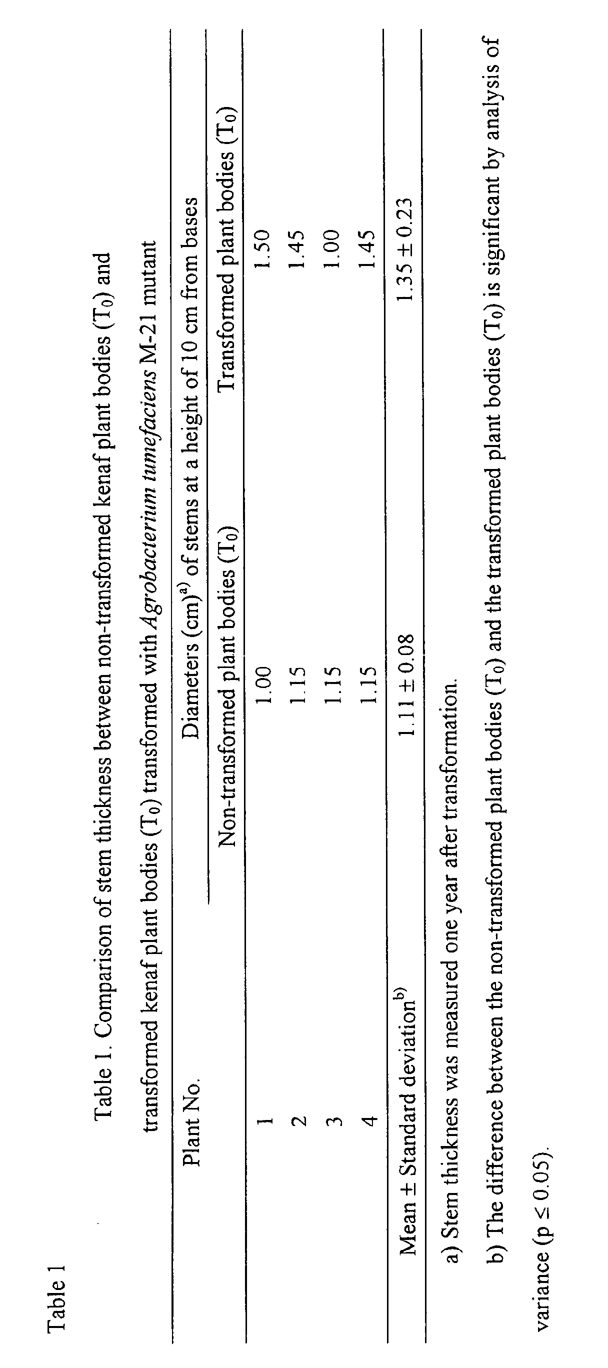

From Figure 2, it is visually found that among the 4 transformed plant bodies (T₀), the stems of 3 transformed plant bodies became thicker than those of the non-transformed plant bodies (T₀). Also, from Table 1, it is found that the differences between the diameters of stems of the non-transformed plant bodies (T₀) and those of the transformed plant bodies (T₀) were statistically significant. In terms of other characteristics and appearance, no differences were observed between the non-transformed plant bodies (T₀) and the transformed plant bodies (T₀).

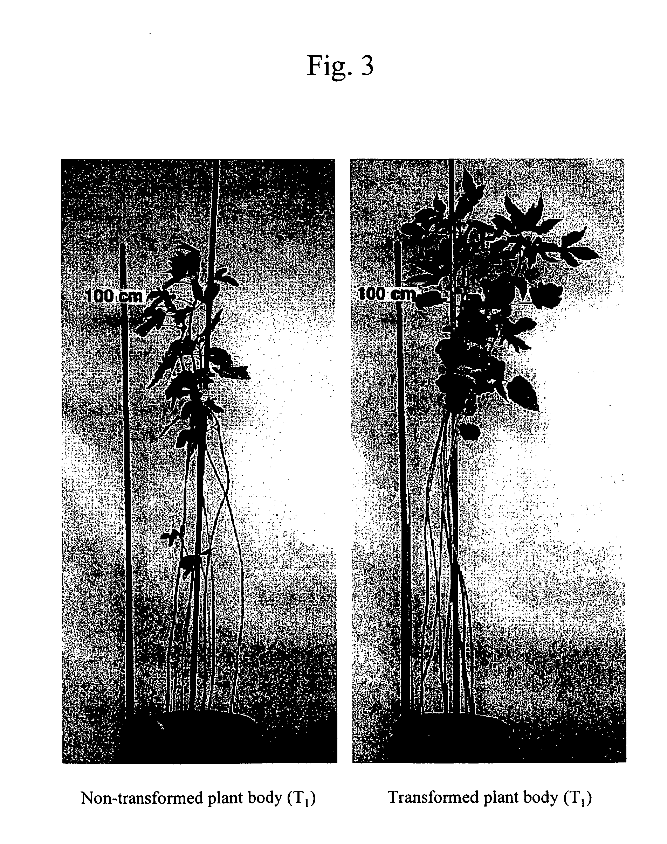

Photographs of the transformed plant bodies (T₁) and the non-transformed plant bodies (T₁), which were taken 2 months after the sowing of seeds, are shown in Figure 3. Moreover, stem thicknesses of the transformed plant bodies (T₁) and those of the non-transformed plant bodies (T₁) measured 3 months after the sowing of seeds are shown in the following Table 2.

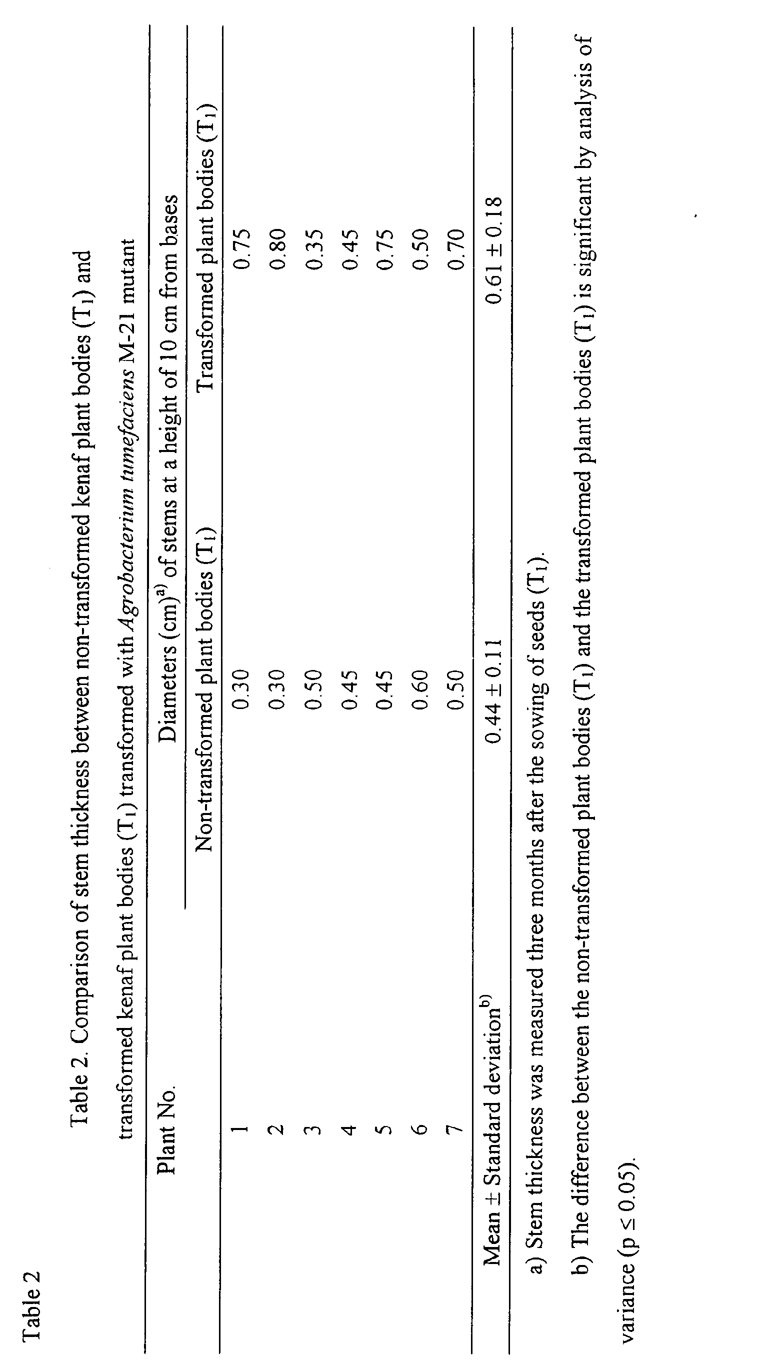

From Figure 3, it is found that the transformed plant bodies (T₁) are taller and greater than the non-transformed plant bodies (T₁) and their stems are thicker than those of the non-transformed plant bodies (T₁), and thus that the transformed plant bodies (T₁) are significantly different from the non-transformed plant bodies (T₁) in terms of appearance. Also, from Table 2, it is found that the diameters of the stems of the transformed plant bodies (T₁) were 1.5 times greater than those of the non-transformed plant bodies (T₁). Thus, the difference in the diameters of the stems was statistically significant. Thus, the phenotypes of the transformed plant bodies (T₁) were inherited from those of the transformed plant bodies (T₀).

### Example 3. Detection of gene derived from Agrobacterium tumefaciens in transformed kenaf plant bodies

### (1) Isolation of genomic DNA from transformed kenaf plant bodies

Genomic DNA was extracted from young leaves (0.05 g) located in the upper portion of each of the aforementioned non-transformed plant bodies (T₀), transformed plant bodies (T₀), non-transformed plant bodies (T₁), and transformed plant bodies (T₁), using Nucleon Phytopure for Plant DNA Extraction Kit (Amersham Biosciences) according to the instructions of the manufacturer. Thereafter, the extracted genomic DNA was treated with RNase at 37°C for 45 minutes, and then with proteinase K at 55°C for 16 hours. Subsequently, the genomic DNA solutions were extracted with phenol, a mixture of phenol and chloroform (1:1), and chloroform. Finally, genomic DNAs were ethanol-precipitated and then dissolved in 100 µl of water. The thus prepared genomic DNA samples were subjected to agarose gel electrophoresis to check the purity and contents of DNA.

### (2) Detection of gene derived from Agrobacterium tumefaciens using nested PCR

In *Agrobacterium tumefaciens* M-21 mutant, a Tn5 is inserted between the base at 1,055 position and the base at 1,056 position of a tryptophan monooxygenase gene in T-DNA region on Ti plasmid (Figure 1). Accordingly, as shown in Figure 4, the following nested PCR primers were designed to amplify a DNA fragment (1,488 bp) that spans the tryptophan monooxygenase gene and Tn5.
Primers for the first PCR:
Forward primer (F1): 5'-AAG ACC TAA TCC GGC GTT TC-3' (SEQ ID NO: 1)
Reverse primer (R1): 5'-TGA GCG TGA TAT TCC CCC TGT-3' (SEQ ID NO: 2)
Primers for the second PCR:
Forward primer (F2): 5'-GAG TAG TCT TTC CGT CTC AG-3' (SEQ ID NO: 3)
Reverse primer (R2): 5'-AGG TTC CGT TCA GGA CGC TA-3' (SEQ ID NO: 4)

In the first PCR, the genomic DNA (approximately 100 ng) as prepared above was added to a reaction mixture with a final volume of 25 µl (50 mM KCl, 10 mM Tris-HCl (pH 8.3), 1.5 mM MgCl₂, 200 µM dNTP, 0.2 µM each primers for the first PCR, and 0.63 unit of Taq DNA polymerase (TAKARA Shuzo)). PCR consisted of: a first denaturation at 94°C for 1 minute; 40 cycles consisting of 94°C, 30 seconds (denaturation), 55°C, 1 minute (annealing), and 72°C, 1 minute (elongation); and a final elongation at 72°C for 7 minutes.

The second PCR was carried out under the same conditions as those in the first PCR with the exceptions that 2 µl of the first PCR reaction solution was used as a template and that primers designed for the second PCR were used. Ten µl of the second PCR reaction solution was subjected to agarose gel (1%) electrophoresis and then stained with ethidium bromide, so as to confirm a PCR product. Also, using the total DNA of *Agrobacterium tumefaciens* M-21 mutant as a template, PCR was carried out under the aforementioned conditions for the second PCR. The obtained PCR product was used as a positive control.

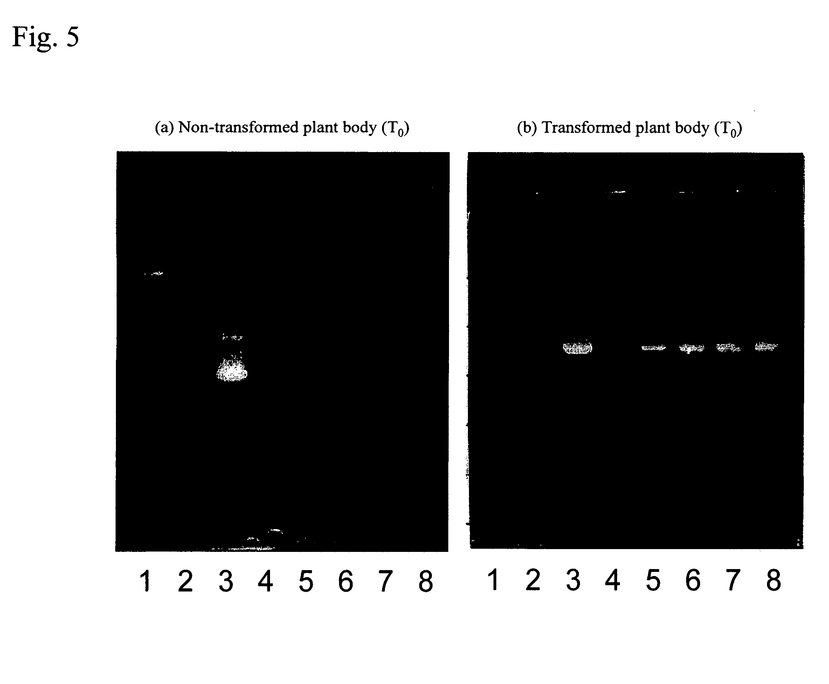

Photographs indicating the results of agarose electrophoresis performed on respective PCR products derived from the non-transformed plant bodies (T₀) and the transformed plant bodies (T₀) are shown in Figure 5. Each of the lanes in panels (a) and (b) in Figure 5 has the following meanings. In panel (a), lane 1: DNA size marker; lane 2: no templates; lane 3: the total DNA of *Agrobacterium tumefaciens* M-21 mutant (positive control); lane 4: no templates; and lanes 5 to 8: each genome DNA derived from different non-transformed plant bodies (T₀). In panel (b), lane 1: DNA size marker; lane 2: no templates; lane 3: the total DNA of *Agrobacterium tumefaciens* M-21 mutant (positive control); lane 4: no templates; and lanes 5 to 8: each genome DNA derived from different transformed plant bodies (T₀).

As shown in Figure 5, approximately 1.5 kb DNA fragment spanning Tn5 and a tryptophan monooxygenase gene in T-DNA region on Ti plasmid of *Agrobacterium* *tumefaciens* M-21 mutant was detected in all the four transformed plant bodies (T₀). In contrast, such a DNA fragment was not detected in the non-transformed plant bodies (T₀).

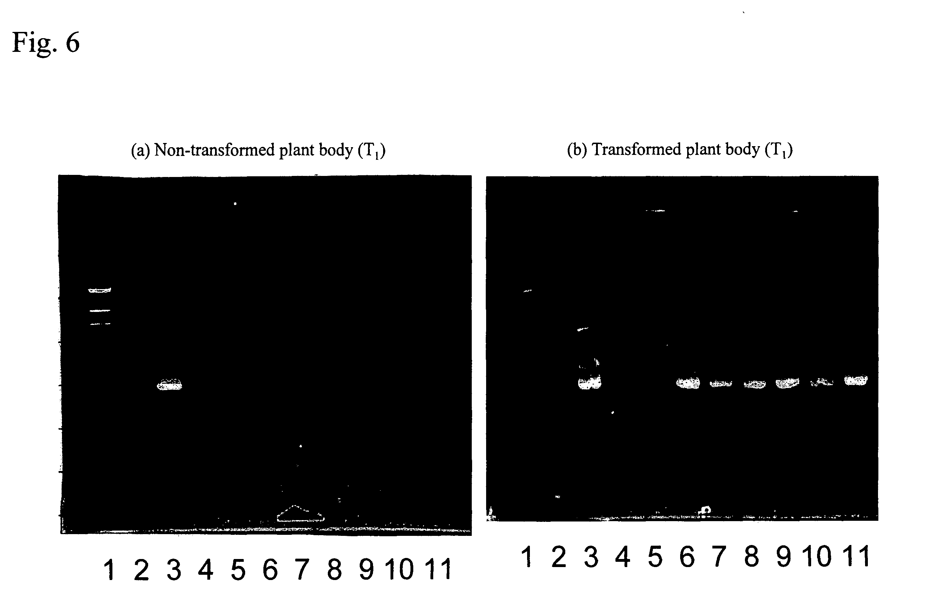

In Figure 6, photographs indicating the results of agarose electrophoresis performed on respective PCR products derived from non-transformed plant bodies (T₁) and transformed plant bodies (T₁) are shown. Each of the lanes in panels (a) and (b) in Figure 6 has the following meanings. In panel (a), lane 1: DNA size marker; lane 2: no templates; lane 3: the total DNA of *Agrobacterium tumefaciens* M-21 mutant (positive control); lane 4: no templates; and lanes 5 to 11: each genome DNA derived from different non-transformed plant bodies (T₁). In panel (b), lane 1: DNA size marker; lane 2: no templates; lane 3: the total DNA of *Agrobacterium tumefaciens* M-21 mutant (positive control); lane 4: no templates; and lanes 5 to 11: each genome DNA derived from different transformed plant bodies (T₁).

As shown in Figure 6, approximately 1.5 kb DNA fragment spanning Tn5 and a tryptophan monooxygenase gene in T-DNA region on Ti plasmid of *Agrobacterium tumefaciens* M-21 mutant was detected in 6 out of 7 transformed plant bodies (T₁). In contrast, such a DNA fragment was not detected in the non-transformed plant bodies (T₁).

From these results, it was found that the phenotypes of the transformed plant bodies (T₀) shown in Example 2 were caused by T-DNA region on Ti plasmid derived from *Agrobacterium tumefaciens* M-21 mutant. It was also found that the genotypes of the transformed plant bodies (T₀) are transmitted to the transformed plant bodies (T₁) at a high frequency.

### Example 4. Verification of the presence of Agrobacterium tumefaciens in transformed kenaf plant bodies

The presence or absence of a surviving *Agrobacterium tumefaciens* M-21 mutant was verified in the transformed plant bodies (T₀ and T₁).

First, a control experiment was carried out. Young leaves in the upper portion of the non-transformed plant bodies (T₀) were used. The leaves were subjected to the following treatments, so as to prepare 4 samples.
Sample 1: non-sterilized;
Sample 2: immersed in 70% ethanol for 3 minutes, followed by washing with water 3 times;
Sample 3: vacuum-infiltrated in an *Agrobacterium tumefaciens* M-21 mutant suspension (1.0 × 10³ cells/ml), followed by washing with water once; and
Sample 4: vacuum-infiltrated in an *Agrobacterium tumefaciens* M-21 mutant suspension (1.0 × 10³ cells/ml), followed by washing with water once, and then immersed in 70% ethanol for 3 minutes, followed by washing with water 3 times.

The term "vacuum-infiltration" is used herein to mean a transformation method comprising immersing a plant in an *Agrobacterium tumefaciens* suspension under a slight negative pressure, so as to allow the suspension to infiltrate into the plant.

All the aforementioned samples were aseptically homogenized in sterilized water. Thereafter, the obtained homogenate was cultured at 28°C for 3 days on LB medium containing kanamycin (50 µg/ml) and rifampicin (10 µg/ml). *Agrobacterium tumefaciens* M-21 mutant can grow under such conditions.

As a result, a large number of colonies appeared on the plates that contained samples 3 and 4. Several colonies appeared on the plate that contained sample 1. However, no colonies were observed on the plate that contained sample 2. The results indicated that *Agrobacterium tumefaciens* M-21 mutant in the leaf tissues was not killed by sterilization of the leaf surface with 70% ethanol.

Subsequently, young leaves in the upper portion of the transformed plant bodies (T₀) and the transformed plant bodies (T₁) were subjected to the same treatment as that for the aforementioned sample 2. Thereafter, the treated leaves were aseptically homogenized in sterilized water. Thereafter, the obtained homogenate was cultured at 28°C for 3 days on LB medium containing kanamycin (50 µg/ml) and rifampicin (10 µg/ml).

As a result, no colonies were observed in the plates each containing samples derived from the transformed plant bodies (T₀) and the transformed plant bodies (T₁).

Seeds obtained from the transformed plant bodies (T₀) were sterilized for 20 minutes in a 1.7% sodium hypochlorite solution. The seeds were then allowed to germinate in an aseptic state. Thereafter, the grown young plants were aseptically homogenized, and the obtained homogenate was then cultured at 28°C for 3 days on LB medium containing kanamycin (50 µg/ml) and rifampicin (10 µg/ml). As a result, no colonies were observed on the plate.

From these results, it was found that no surviving *Agrobacterium tumefaciens* M-21 mutants were present either in the transformed plant bodies (T₀) or the transformed plant bodies (T₁). It was also found that the T-DNA region derived from *Agrobacterium tumefaciens* M-21 mutant detected in Example 3 was derived from the T-DNA region incorporated into the genomes of the transformed plant bodies (T₀) or those of the transformed plant bodies (T₁).

All publications, patents and patent applications cited herein are incorporated herein by reference in their entirety.

### Industrial Applicability

The present invention provides an *in planta* transformation method of kenaf plants for simply and efficiently obtaining a kenaf plant transformant. Thus, according to the present invention, economically and agriculturally valuable kenaf plants are obtained.

## Claims

1. An *in planta* transformation method of kenaf plants, which is **characterized in that** wounds on the axillary buds of a kenaf plant are inoculated with *Agrobacterium tumefaciens*.

2. The *in planta* transformation method of kenaf plants according to claim 1, which comprises a step of cutting off the upper part of stem of a kenaf plant and allowing the axillary buds to appear.

3. The *in planta* transformation method of kenaf plants according to claim 1 or 2, which comprises a step of wounding said axillary buds.

4. The *in planta* transformation method of kenaf plants according to any one of claims 1 to 3, wherein said wound is a perforated hole.

5. The *in planta* transformation method of kenaf plants according to any one of claims 1 to 4, wherein *Agrobacterium tumefaciens* to be inoculated is an *Agrobacterium tumefaciens* M-21 mutant.

6. The *in planta* transformation method of kenaf plants according to any one of claims 1 to 5, wherein said inoculation is carried out by applying an *Agrobacterium tumefaciens* suspension to the wound.


SEQUENCE LISTING

<110> Kumiai Chemical Industry Co., Ltd.
      Mineo, Kojima

<120> In planta transformation method of kenaf plants by
      Agrobacterium tumefaciens

<130> PH-1890-PCT

<140> PCT/JP2003/014148
<141> 2003-11-06

<150> JP 2002-340992
<151> 2002-11-25

<160> 4

<170> PatentIn Ver. 2.0

<210> 1
<211> 20
<212> DNA
<213> Artificial Sequence

<220>
<223> Description of Artificial Sequence:primer

<400> 1
aagacctaat ccggcgtttc                                              20

<210> 2
<211> 21
<212> DNA
<213> Artificial Sequence

<220>
<223> Description of Artificial Sequence:primer

<400> 2
tgagcgtgat attccccctg t                                                21

<210> 3
<211> 20
<212> DNA
<213> Artificial Sequence

<220>
<223> Description of Artificial Sequence:primer

<400> 3
gagtagtctt tccgtctcag                                                  20

<210> 4
<211> 20
<212> DNA
<213> Artificial Sequence

<220>
<223> Description of Artificial Sequence:primer

<400> 4
aggttccgtt caggacgcta                                                  20